# EUROPEAN PATENT APPLICATION

(11) **EP 2 527 369 A2**
(43) Date of publication of application: **28.11.2012**
(21) Application number: 12178587.7
(22) Date of filing: 15.09.2008
(51) Int. Cl.: C07K 16/18, A61P 25/28, G01N 33/577, A61K 39/395

(54) **Monoclonal amyloid beta (abeta)-specific antibody and uses thereof**

(30) Priority: 13.09.2007 US 993749 P
(62) Divisional of application: 08802218.1
(71) Applicant: University Of Zurich Prorektorat Forschung, 8006 Zurich (CH)
(72) Inventor: Nitsch, Roger, 8126 Zumikon (CH)
(74) Representative: Steinecke, Peter

(57) **Abstract**

A novel monoclonal antibody against beta-amyloid (Abeta) peptide is provided with unique immunological and biological properties useful in the immunotherapy of Alzheimer's disease. In addition, pharmaceutical compositions and kits comprising such antibody and mimics thereof in the treatment of neurological disorders such as Alzheimer's disease are described.

## Description

### Field of the invention

The present invention relates to antibodies as well as fragments, derivatives and variants thereof that recognize a unique epitope of amyloid beta (Abeta) peptide. In addition, the present invention relates to compositions comprising such antibodies and mimics thereof, and to methods of screening for novel binding molecules, which may or may not be antibodies and drugs in the treatment of neurological disorders such as Alzheimer's disease and amyloidoses involving beta-amyloid.

### Background of the invention

Alzheimer's disease (AD), the most common cause of dementia, is an age-related neurodegenerative disorder that is characterized by progressive cognitive deficits, such as memory loss and a decline in mental abilities. An elevated abnormal level of the beta-amyloid peptide (Abeta) in the brain is the key step in the pathogenesis of AD (Selkoe, J. Alzheimers Dis. 3 (2001), 75-80; Selkoe, Nature 399 (1999), 6738 suppl. A23-A31; Hardy and Selkoe, Science 297 (2002), 353-356). Abeta peptide accumulation is associated with the formation of neurofibrillary tangles (Lewis et al., Science 293 (2001), 1487-1491; Gotz et al., Science 293 (2001), 1491-1495) with impaired synaptic functions and the loss of neurons. Therefore, a major emphasis of AD therapy has currently been on the removal of Abeta from the affected brains.

Both active and passive immunization approaches were effective in reducing the brain Abeta levels in human patients and in AD mouse models expressing familial mutations of the amyloid precursor protein (APP) resulting in the accumulation of Abeta and age-dependent amyloid plaque deposition (Nicoll et al. A case report. Nat. Med. 9 (2003), 448-452; DeMattos et al., Proc. Natl. Acad. Sci. USA 98 (2001), 8850-8855; Bard et al., Nat. Med. 6 (2000), 916-919; Schenk et al., Nature 400 (1999), 173-177; Mohajeri et al., J. Biol. Chem. 277 (2002), 33012-33017). In addition, immunization with the Abeta peptide or passive transfer of anti-Abeta monoclonal antibodies exerted beneficial effects on cognitive performances oftransgenic mouse models of AD (Morgan et al., Nature 408 (2000), 982-985; Janus et al., Nature 408 (2000), 979-982) and in human subjects (Hock et al., Neuron 38 (2000), 547-554), providing further support for the validity of Abeta-directed immunotherapy. Furthermore, it was recently shown that passive anti-Abeta immunotherapy protected the neurons against apoptotic stimuli in an AD mouse model (Mohajeri et al., J. Biol. Chem. 277 (2002), 33012-33017).

Mechanism implicated in the reduction of brain Abeta by antibodies may include the action as "peripheral sink" whereby Abeta-specific antibody molecules present in the circulation are believed to sequester the Abeta peptide in peripheral compartments, thereby reducing cerebral Abeta levels without entering the brain (DeMattos et al., Proc. Natl. Acad. Sci. USA 98 (2001), 8850-8855). Alternatively, antibodies may penetrate into the brain, bind the Abeta peptide and interfere with its aggregation or promote the dissolution of beta-amyloid fibrils and plaques (Bard et al., Nat. Med. 6 (2000), 916-919; Bacskai et al., Nat. Med. 7 (2001), 369-372; Frenkel et al., Proc. Nat. Acad. Sci. USA 97 (2000), 11455-11459). Upon opsonization of beta-amyloid plaques by antibodies, these complexes can be phagocytosed by brain microglial cells, providing further routes for Abeta clearance (Bard et al., Nat. Med. 6 (2000), 916-919; Bacskai et al. Nat. Med. 7 (2001), 369-372; Bacskai et al., J. Neurosci. 22 (2002), 7873-7878).

Regardless of the high potential, Abeta-directed approaches for active as well as passive immunotherapy can produce varying effects with respect to their efficacy towards specific therapeutic endpoints and can be associated with adverse events such as autoimmune disease, meningoencephalitis, increased cerebral amyloid angiopathy and the induction of cerebral hemorrhages in preclinical mouse models of AD or in human (Pfeifer et al., Science 298 (2002), 1379; Furlan et al., Brain 126 (2003), 285-291; Wilcock et al., J. Neuroinflammation 1:24 (2004); Lee at al., FEBS Lett. 579 (2005), 2564-8; Wilcock et al., Neuroscience 144 (2007), 950-960; Schenk, Nat. Rev. Neurosci. 3 (2002), 824-828; Orgogozo et al, Neurology 61 (2003), 46-54). Despite intense research, the exact mechanisms of action of anti-Abeta immunotherapy underlying the desired therapeutic effects as well as the ones associated with the various side effects remain controversial.

Thus, there is still a need of providing agents which are specific for Abeta and which display bioactivities which make them suitable for passive immunization in vivo.

### Summary of the invention

The present invention is directed to antibodies and antigen-binding fragments and similar antigen binding molecules which
(a) are capable of recognizing a unique epitope essentially consisting of the amino acid sequence of amino acids 29-40 of Abeta;
(b) display a high and substantially identical affinity for both Abetal-40 and Abetal-42;
(c) display a high affinity for aggregated forms of Abeta; and
(d) do not substantially recognize amyloid precursor protein (APP) in vivo.

Furthermore, the present invention relates to compositions comprising said antibodies and to immunotherapeutic and immunodiagnostic methods using the same.

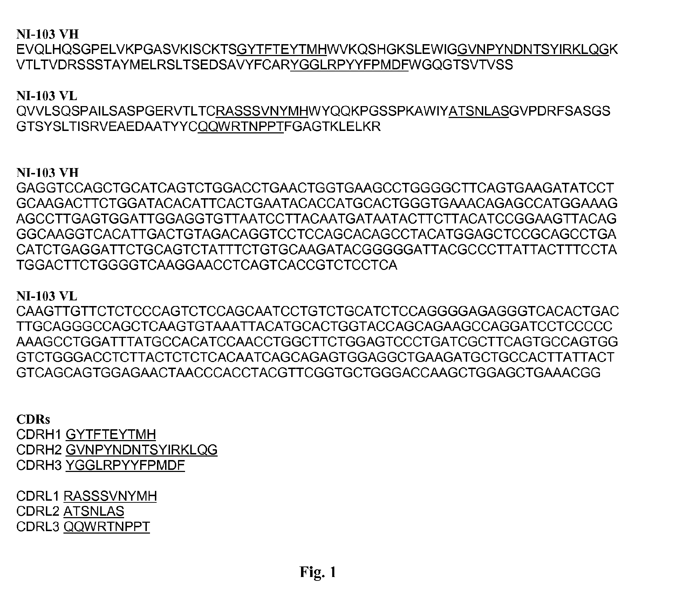

A particularly preferred embodiment is a monoclonal antibody or antigen-binding fragment thereof which demonstrates the immunological binding characteristics of the antibody characterized by the variable regions V_{H} and/or V_{L} as set forth in Figure 1, infra.

Alternatively, the antibody is a human, humanized, xenogeneic, or a chimeric human-murine antibody, the latter being particularly useful for diagnostic methods and studies in animals. Compositions including the antibody or active fragments thereof, or agonists and cognate molecules, or alternately, antagonists of the same, and methods of use of such compositions in the prevention, diagnosis or treatment of a disease using these compositions are also included, wherein an effective amount of the composition is administered to a patient in need of such treatment.

The antigen-binding fragment of the monoclonal antibody can be a single chain Fv fragment, an F(ab') fragment, an F(ab) fragment, and an F(ab')₂ fragment, or any other antigen-binding fragment. In a specific embodiment, infra, the monoclonal antibody or fragment thereof is a murine IgG isotype antibody.

Naturally, the present invention extends to the hybridoma that produces the monoclonal antibody having the distinct and unique characteristics as defined below.

The present invention also relates to polynucleotides encoding at least a variable region of an immunoglobulin chain of the antibody of the invention. Preferably, said variable region comprises at least one complementarity determining region (CDR) of the V_{H} and/or V_{L} of the variable region as set forth in Figure 1, infra.

Accordingly, the present invention also encompasses vectors comprising said polynucleotides and host cells transformed therewith as well as their use for the production of an antibody and equivalent binding molecules which are specific for Abeta that is indicative and/or causative for a disorder, in particular for a disorder of the brain such as Alzheimer's.

The antibody, immunoglobulin chain(s), binding fragments thereof and ligands other than Abeta binding to said antibody can be used in pharmaceutical and diagnostic compositions for immunotherapy and diagnosis, respectively, as well as in extracorporeal immunoadsorption techniques such as therapeutic apheresis procedures including plasmapheresis/plasma exchange. The use of the foregoing compositions in the preparation of a medicament is however preferred.

Hence, it is a particular object of the present invention to provide methods for treating or preventing a neurological disorder or amyloidosis characterized by abnormal accumulation and/or deposition of a protein in the central nervous system or peripheral tissues without interfering with the physiologic precursor of the respective protein. The methods comprise administering an effective concentration of an antibody or antibody derivative to the subject where the antibody binds to the pathological Abeta peptide with a substantially higher affinity than to the physiological cellular form of the amyloid precursor protein. In a preferred embodiment, the present invention provides methods for treating or preventing or slowing the onset of diseases associated with the accumulation and deposition of the amyloid beta peptide in a subject, such as Alzheimer's disease, Down's syndrome, mild cognitive impairment, cerebral amyloid angiopathy, Lewy body dementia, vascular dementia, mixed dementia, multi-infarct dementia, hereditary cerebral hemorrhage with amyloidosis Dutch type and Icelandic type, glaucoma and inclusion body myositis.

Further embodiments of the present invention will be apparent from the description that follows.

### Brief description of the drawings

- **Fig. 1:**: Amino acid sequences of the variable light chain and heavy chain of antibody NI-103 with complementarity determining regions (CDRs) underlined as well as the corresponding DNA sequences. VL, variable light chain; VH, variable heavy chain; CDRL, CDR of the variable light chain; CDRH, CDR of the variable heavy chain. These sequences were expressed as IgG2a antibody, and found to bind in TAPIR-assay on human and mouse FFPE tissue as described in US patent application US 2006/0240485 A1, the disclosure content of which is incorporated herein by reference.

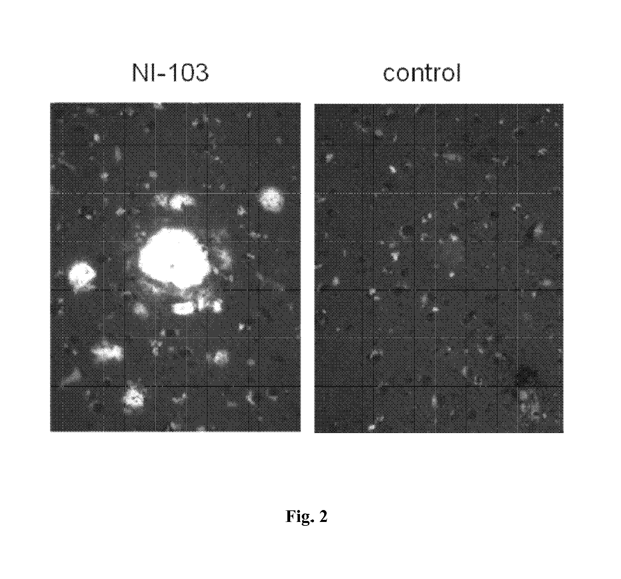

- **Fig. 2:**: Immunohistochemical staining with NI-103 antibody of brain beta amyloid plaques in brain sections obtained from patients with pathologically confirmed Alzheimer's disease.

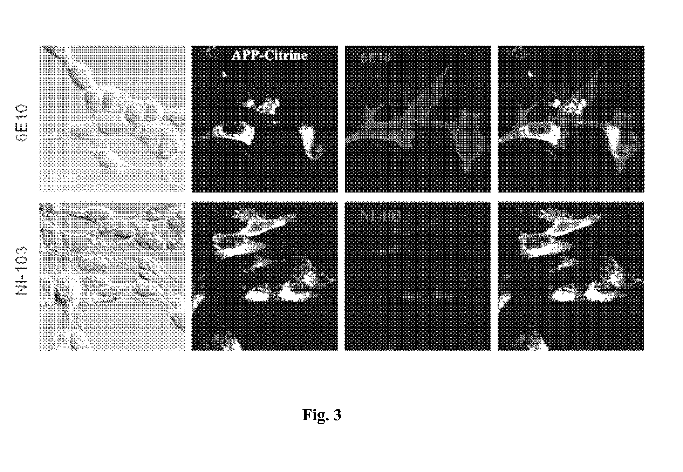

- **Fig. 3:**: Absent cross-reactivity of NI-103 antibody to cellular full-length APP or with any of its physiological derivatives occurring in cultured cells. In contrast to the control antibody (6E10) that binds to cell-surface APP, binding of NI-103 to full-length APP present at cellular surfaces is absent. These data demonstrate absent cross-reactivity of NI-103 to physiological, cellular full-length APP

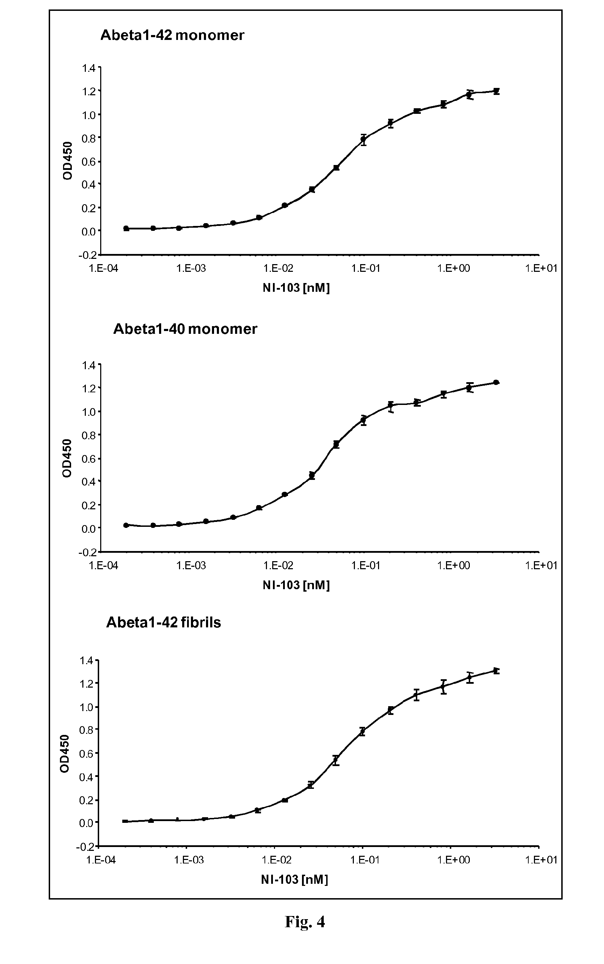

- **Fig. 4:**: NI-103 antibody binds to synthetic monomeric Abetal-40 peptide, synthetic monomeric Abetal-42 peptide and Abetal-42 fibrils with high and substantially identical affinity. Synthetic Abetal-40 or Abetal-42 monomers or Abetal-42 fibril preparations were coated onto ELISA plates at equal coating densities and incubated with the indicated concentrations of NI-103 antibody. High affinity binding with substantially identical EC50 was observed for all Abeta species assayed.

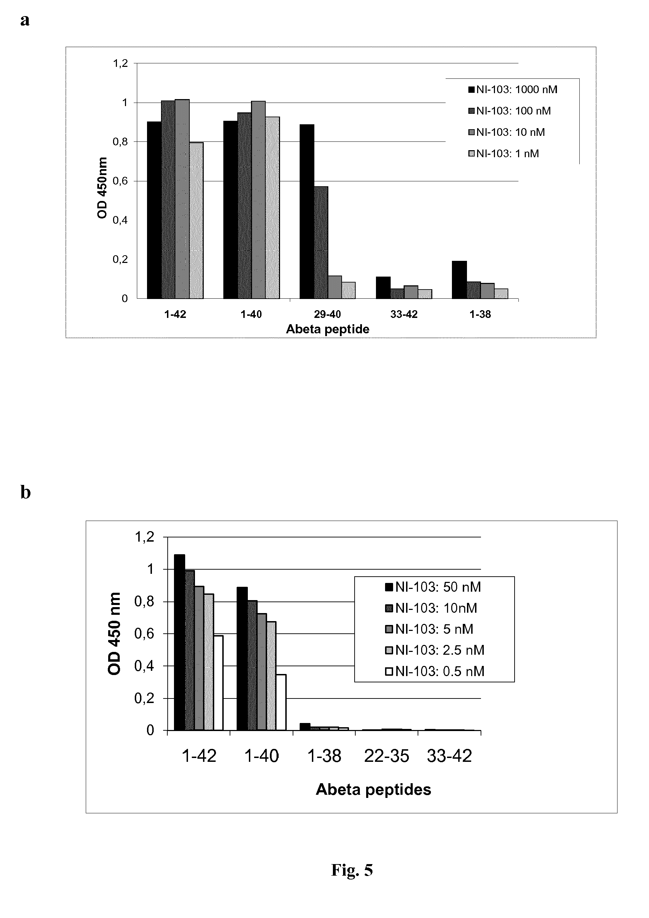

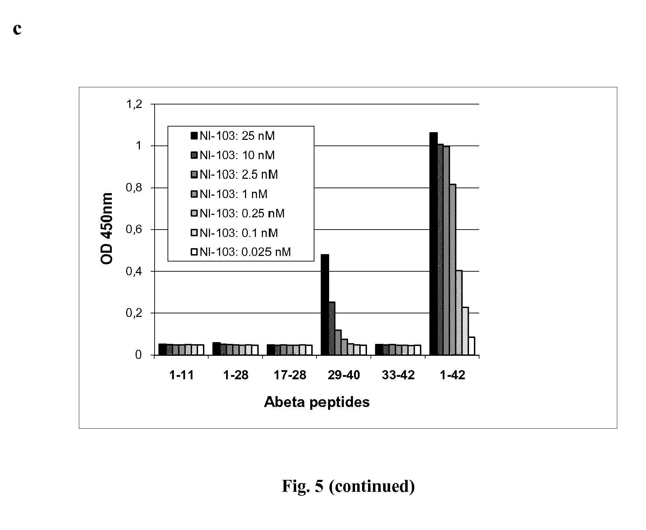

- **Fig. 5:**: Epitope mapping of antibody NI-103 by ELISA reveals a C-terminal epitope. Abeta pepties and fragments were coated on ELISA plates at 1 µg/ml concentration. Binding of NI-103 was determined at the indicated concentrations.

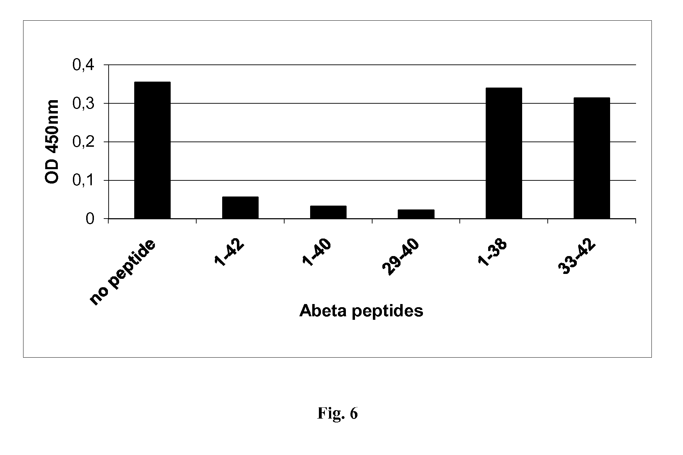

- **Fig. 6:**: NI-103 antibody epitope mapping by competition ELISA reveals a C-terminal epitope including amino acids 29-40. The binding of 0.5 nM NI-103 to Abetal-42 coated on the ELISA plate was completely blocked by pre-incubation of NI-103 with Abetal-40, Abeta1-42 and Abeta29-40. Abeta fragments consisting of amino acids 1-38 and 33-42 did not compete for binding.

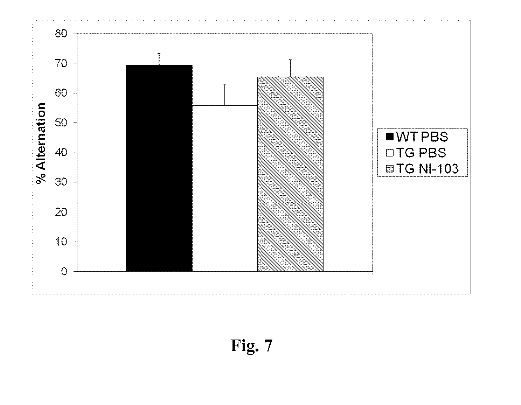

- **Fig. 7:**: Passive immunization of APPswe/PS1 mice with antibody NI-103 is associated with significant improvement in working memory, as shown by the increased frequency of spontaneous alternation, p=0.01.

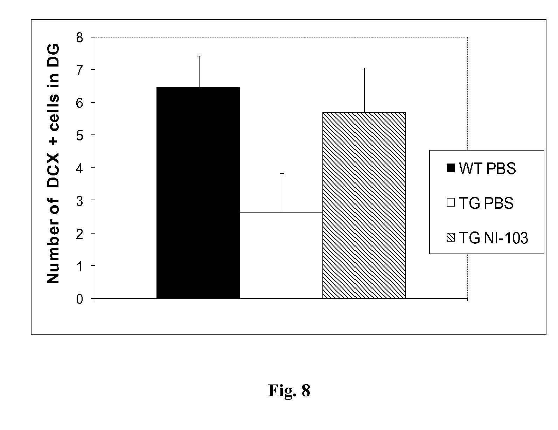

- **Fig. 8:**: Systemic treatment of APPswe/PS1 mice with NI-103 antibody for three months restores the numbers of DCX+ neurons to almost wide type levels.

### Detailed description of the invention

The present invention provides antibodies and binding molecules which are capable of recognizing both, the C-terminus of Abeta₄₀ and Abeta₄₂; see also Example 3. In a preferred embodiment of the present invention, the antibody or equivalent binding molecule recognizes a unique epitope that is contained within and essentially consists of, respectively, the amino acid sequence GAIIGLMVGGVV (SEQ ID NO: 1). Most preferably, said antibody is a monoclonal antibody.

In particular, antibodies and antigen-binding fragments thereof are provided, which demonstrate the immunological binding characteristics and/or biological properties as outlined for the antibody illustrated below and in the Examples. Where present, the term "immunological binding characteristics," or other binding characteristics of an antibody with an antigen, in all of its grammatical forms, refers to the specificity, affinity, cross-reactivity, and other binding characteristics of an antibody.

Naturally, the present invention extends to the antibody producing cell lines and recombinant cells as well. Thus, the present invention advantageously provides recombinant means and indefinitely prolonged cells as a source of a monoclonal antibody of the present invention. The present invention further relates to diagnostic assays and kits that comprise the antibody of the present invention or an equivalent binding molecule and to therapeutic methods based thereon.

The immunological binding characteristics and bioactivities of the monoclonal antibody of the present invention, denoted NI-103 in the examples are substantially the same as those of antibody 22C4 described by Mohajeri et al. in J. Biol. Chem. 277 (2002), 33012-33017 and Neurodegenerative Dis. 1 (2004), 160-167, the disclosure content of both being incorporated herein by reference. However, while those publications describe some of the immunological and biological features of the antibody of the present invention, especially its binding to the C-terminus of the Abeta peptide, its ability to mediate the uptake of fibrillar Abeta by microglia and to reduce Abeta levels in vivo, the present invention for the first time enables the provision of such an antibody and reliable means for its recombinant production, in particular the amino acid sequences of the antibody's variable light and heavy chains including the complementarity determining regions (CDRs).

Hitherto no reliable source of an antibody according to the present invention was available, for example because of loss of specificity or change of specificity of a monoclonal antibody due to the hybridoma cell line producing the initial antibody not being derived from a single cell line. Thus, a mixture of two or more different hybridomas will produce a mixture of two or more monoclonal antibodies with different specificity. The ratio of the cells within the culture and thereby the ratio of the different monoclonal antibodies produced by them may vary during cultivation. Also, a particular hybridoma producing the antibody with the desired specificity might be lost from the mixture if the other hybridoma cells have an evolutionary advantage. A mixture of hybridomas in a culture can also result from mutations in certain cells leading to shifting specificities of the antibodies produced.

In addition, though the antigen used for immunization and generation of the monoclonal antibodies described in Mohajeri et al., Neurodegenerative Dis. 1 (2004), 160-167 are indicated, it turned out that the antigen used to generate inter alia monoclonal antibody 22C4 is only weakly immunogenic. For this reason, up to about 30 mice had to be immunized and screened in order to obtain antibodies against this antigen. Thus, it was a mere stroke of luck to arrive at the 22C4 antibody since it could and cannot be expected to arrive at such antibody twice if it is tried to raise monoclonal antibodies against this antigen again. This is all the more true in view of the fact that the monoclonal antibodies obtained with this particular antigen were predominantly different in nature such as antibody 22D4 raised against the same antigen but being for example completely ineffective in facilitating Abeta uptake by microglia cells; see Mohajeri et al. (2004), supra, in Figure 1 and 2, as well as at page 164, left column, first paragraph.

This confirms the experience often encountered that knowledge of an epitope or antigen used to generate a monoclonal antibody is insufficient for making the original antibody available, even if suitable in vitro test systems for screening are used.

Hence, the provision of the amino acid sequences of the variable light and heavy chains of the antibody of the present invention for the first time enables the person skilled in the art to design and produce functionally equivalent antibodies, for example by adapting the antigen-binding side of antibody NI-103.

Furthermore, the appended examples performed in accordance with the present invention revealed further unique immunological and biological activities of the antibody of the invention. For example, the antibody of the present invention may be distinguished from other anti-Abeta antibodies by its ability to bind to the C-terminus of both the Abetal-40 and Abetal-42 peptides with substantially the same binding affinity. Previous anti-Abeta antibodies that target the C-terminus of Abeta have been reported to have a substantial preference for the 40, 42 or 43 amino acid variant Abeta peptide.

In addition, as shown in example 4 the antibody of the present invention is therapeutically active by improving cognitive behavior and rescuing the number of immature neurons in a transgenic mouse model of Alzheimer's disease. Thus, the antibody of the present invention as well as equivalent binding molecules may be characterized by their biological activity of protecting CNS neurons from increased vulnerability associated with Abeta related toxicity and more particularly for being capable of rescuing the number of immature DCX positive neurons in APP transgenic mice.

As mentioned, the antibody of the present invention is preferably also capable of mediating the uptake of fibrillar Abeta by microglia and reducing Abeta levels in the brain; see Mohajeri et al. (2004), supra, with respect to the results of the 22C4 antibody in Figure 3. In this context, the antibody of the present invention is preferably capable of binding to amyloid plaques in the brain as well. In addition, the antibody of the present invention advantageously capable of protecting neurons from Abeta related toxicity as described for the 22C4 antibody in Mohajeri et al. (2002), supra.

Thus, the present invention generally relates to any antibody, in particular monoclonal antibody, antigen-binding fragments thereof and equivalent binding molecules which demonstrate the immunological binding characteristics and preferably biological activity of the NI-103 antibody of the present invention as described above and demonstrated in the examples.

A "binding molecule" as used in the context of the present invention relates primarily to antibodies, and fragments thereof, but may also refer to other non-antibody molecules that bind to Abeta and exhibit the functional properties of the NI-103 antibody of the present invention including but not limited to hormones, receptors, ligands, major histocompatibility complex (MHC) molecules, chaperones such as heat shock proteins (HSPs) as well as cell-cell adhesion molecules such as members of the cadherin, intergrin, C-type lectin, immunoglobulin (Ig) superfamilies and in particular designed ankyrin repeat proteins (DARPins) which are a promising class of non-immunoglobulin proteins that can offer advantages over antibodies for target binding; see for review, e.g., Stumpp and Amstutz, Curr. Opin. Drug Discov. Devel. 10 (2007), 153-159, and references cited therein. Thus, for the sake of clarity only and without restricting the scope of the present invention most of the following embodiments are discussed with respect to antibodies and antibody-like molecules which represent the preferred binding molecules for the development of therapeutic and diagnostic agents.

Means and methods for the recombinant production of binding molecules, in particular antibodies and mimics thereof as well as methods of screening for competing binding molecules, which may or may not be antibodies, are known in the art and are summarized, for example, in international application W02006/103116 with respect to antibodies against beta-amyloid and the treatment/diagnosis of Alzheimer's disease, the disclosure content of which is incorporated herein by reference for this purpose of antibody engineering and administration for therapeutic or diagnostic applications.

As demonstrated in the Examples the binding molecule of the present invention, in particular an antibody has several advantageous biological properties one or more of which have been accomplished by the present invention for the first time, i.e. it is capable of
(i) binding to the C-terminus of both the Abeta1-40 and Abeta1-42 peptide with a high and substantially identical affinity;
(ii) displaying a high affinity for aggregated and monomeric forms of Abeta;
(iii) not substantially recognizing amyloid precursor protein (APP) in vivo;
(iv) mediating the uptake of fibrillar Abeta by microglia;
(v) binding beta-amyloid plaques;
(vi) removing beta-amyloid plaques in the brain and/or preventing the formation of amyloid plaques in the brain;
(vii) decreasing Abeta toxicity and associated vulnerability of neurons to excitotoxic events produced by seizures;
(viii) crossing the blood brain barrier, for example at the site of the pathological event;
(ix) rescuing the number of immature DCX positive neurons in APP transgenic mice; and/or
(x) substantially restoring normal behavior.

Preferably, the binding molecule of the present invention has at least two, more preferably three, still more preferably four, particularly preferred five, most preferred six and advantageously seven or even all those properties (i) to (x).

Furthermore, the binding molecule of the present invention has a preferential binding affinity to Abeta over the APP by a factor of at least two, preferably at least 5, usually more than by a factor of 10, particularly preferred by a factor of 50 and even more preferred higher than 100. Furthermore, the relative K_{D} of the binding molecule, e.g., antibody is preferably at least 10-fold less, more preferably at least 100-fold less than the K_{D} for binding that antibody to other ligands or to the APP.

Typically, the antibody of the present invention comprises in its epitope binding domain at least one complementarity determining region (CDR) of the V_{H} and/or V_{L} chain of the variable region comprising the amino acid sequences as depicted in Figure 1 and set forth in SEQ ID NOS. 6 to 11, respectively.

In one embodiment, the antibody of the present invention is any one of antibody comprising an amino acid sequence of the V_{H} and/or V_{L} region as depicted in Fig. 1. Alternatively, the antibody of the present invention is an antibody or antigen-binding fragment thereof, which competes for binding to the Abeta peptide with the antibody having the V_{H} and V_{L} region as depicted in Fig. 1. Those antibodies may be murine, however, humanized, xenogeneic, or chimeric human-murine antibodies being preferred, in particular for therapeutic applications. However, for diagnostic uses and research in general murine antibodies are suitable as well. An antigen-binding fragment of the antibody can be, for example, a single chain Fv fragment (scFv), a F(ab') fragment, a F(ab) fragment, and an F(ab')₂ fragment.

For some applications only the variable regions of the antibodies are required, which can be obtained by treating the antibody with suitable reagents so as to generate Fab', Fab, or F(ab")₂ portions. Such fragments are sufficient for use, for example, in immunodiagnostic procedures involving coupling the immunospecific portions of immunoglobulins to detecting reagents such as radioisotopes.

In accordance with the above, the present invention also relates to a polynucleotide encoding the binding molecule of the present invention, in case of the antibody preferably at least the binding domain or variable region of an immunoglobulin chain of the antibody described above. Typically, said variable region encoded by the polynucleotide comprises at least one complementarity determining region (CDR) of the V_{H} and/or V_{L} of the variable region of the said antibody. The person skilled in the art knows that each variable domain (the heavy chain V_{H} and light chain V_{L}) of an antibody comprises three hypervariable regions, sometimes called complementarity determining regions or "CDRs" flanked by four relatively conserved framework regions or "FRs" and refer to the amino acid residues of an antibody which are responsible for antigen-binding. The hypervariable regions or CDRs of the human IgG subtype of antibody comprise amino acid residues from residues 24-34 (L1), 50-56 (L2) and 89-97 (L3) in the light chain variable domain and 31-35 (H1), 50-65 (H2) and 95-102 (H3) in the heavy chain variable domain as described by Kabat et al., Sequences of Proteins of Immunological Interest, 5th Ed Public Health Service, National Institutes of Health, Bethesda, Md (1991) and/or those residues from a hypervariable loop, i.e. residues 26-32 (L1), 50-52 (L2) and 91-96 (L3) in the light chain variable domain and 26-32 (H1), 53-55 (H2) and 96-101 (H3) in the heavy chain variable domain as described by Chothia et al., J. MoI. Biol. 196 (1987), 901-917. Framework or FR residues are those variable domain residues other than and bracketing the hypervariable regions. The term "specific binding" and "high affinity", respectively, refers to antibody binding to a predetermined antigen, i.e. the Abeta epitope defined above. Typically, the antibody binds with a dissociation constant (K_{D}) of 10⁻⁷ M or less, and binds to the predetermined antigen with a K_{D} that is at least twofold less than its K_{D} for binding to a nonspecific antigen (e.g., BSA, casein, or any other specified polypeptide) other than the predetermined antigen. The phrases "an antibody recognizing an antigen" and "an antibody specific for an antigen" are used interchangeably herein with the term "an antibody which binds specifically to an antigen". As used herein "highly specific" binding means that the relative K_{D} of the antibody for the specific Abeta epitope is at least 10-fold less than the K_{D} for binding that antibody to other ligands or to APP.

The affinity or avidity of an antibody for an antigen can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. The measured affinity of a particular antibody-antigen interaction can vary if measured under different conditions, e.g., salt concentration, pH. Thus, measurements of affinity and other antigen-binding parameters, e.g., K sub D, IC50, are preferably made with standardized solutions of antibody and antigen, and a standardized buffer.

The person skilled in the art will readily appreciate that the variable domain of the antibody having the above-described variable domain can be used for the construction of other polypeptides or antibodies of desired specificity and biological function. Thus, the present invention also encompasses polypeptides and antibodies comprising at least one CDR of the above-described variable domain and which advantageously have substantially the same or similar binding properties as the antibody described in the appended examples. The person skilled in the art will readily appreciate that using the variable domains or CDRs described herein antibodies can be constructed according to methods known in the art, e.g., as described in European patent applications EP 0 451 216 Al and EP 0 549 581 A1. Furthermore, the person skilled in the art knows that binding affinity may be enhanced by making amino acid substitutions within the CDRs or within the hypervariable loops (Chothia and Lesk, J. Mol. Biol. 196 (1987), 901-917) which partially overlap with the CDRs as defined by Kabat. Thus, the present invention also relates to antibodies wherein one or more of the mentioned CDRs comprise one or more, preferably not more than two amino acid substitutions. Preferably, the antibody of the invention comprises in one or both of its immunoglobulin chains two or all three CDRs of the variable regions as set forth in Fig. 1.

The polynucleotide of the invention encoding the above described antibody may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions.

Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers, and/or naturally associated or heterologous promoter regions.

In this respect, the person skilled in the art will readily appreciate that the polynucleotides encoding at least the variable domain of the light and/or heavy chain may encode the variable domains of both immunoglobulin chains or only one. Likewise, said polynucleotides may be under the control of the same promoter or may be separately controlled for expression. Possible regulatory elements permitting expression in prokaryotic host cells comprise, e.g., the P_{L}, lac, trp or tac promoter in E. coli, and examples for regulatory elements permitting expression in eukaryotic host cells are the AOX1 or GAL1 promoter in yeast or the CMV-, SV40-, RSV-promoter, CMV-enhancer, SV40-enhancer or a globin intron in mammalian and other animal cells.

Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including a C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (Invitrogen), or pSPORT1 (GIBCO BRL).

Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the immunoglobulin light chains, heavy chains, light/heavy chain dimers or intact antibodies, binding fragments or other immunoglobulin forms may follow; see, Beychok, Cells of Immunoglobulin Synthesis, Academic Press, N.Y., (1979).

Furthermore, the present invention relates to vectors, particularly plasmids, cosmids, viruses and bacteriophages used conventionally in genetic engineering that comprise a polynucleotide encoding a variable domain of an immunoglobulin chain of an antibody of the invention; optionally in combination with a polynucleotide of the invention that encodes the variable domain of the other immunoglobulin chain of the antibody of the invention. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Alternatively, the polynucleotides and vectors of the invention can be reconstituted into liposomes for delivery to target cells. The vectors containing the polynucleotides of the invention (e.g., the heavy and/or light variable domain(s) of the immunoglobulin chains encoding sequences and expression control sequences) can be transferred into the host cell by well known methods, which vary depending on the type of cellular host. For example, calcium chloride transfection is commonly utilized for prokaryotic cells, whereas calcium phosphate treatment or electroporation may be used for other cellular hosts; see Sambrook, supra.

The present invention furthermore relates to host cells transformed with a polynucleotide or vector of the invention. Said host cell may be a prokaryotic or eukaryotic cell. The polynucleotide or vector of the invention which is present in the host cell may either be integrated into the genome of the host cell or it may be maintained extrachromosomally. The host cell can be any prokaryotic or eukaryotic cell, such as a bacterial, insect, fungal, plant, animal or human cell. Preferred fungal cells are, for example, those of the genus Saccharomyces, in particular those of the species S. cerevisiae. The term "prokaryotic" is meant to include all bacteria which can be transformed or transfected with a DNA or RNA molecules for the expression of an antibody of the invention or the corresponding immunoglobulin chains. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells, most preferably HEK 293, NSO and CHO cells. Depending upon the host employed in a recombinant production procedure, the antibodies or immunoglobulin chains encoded by the polynucleotide of the present invention may be glycosylated or may be non-glycosylated. Antibodies of the invention or the corresponding immunoglobulin chains may also include an initial methionine amino acid residue. A polynucleotide of the invention can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, Molecular Cloning: A Laboratory Manual, Cold Spring Harbor Laboratory, Cold Spring Harbor, NY, 1989). The genetic constructs and methods described therein can be utilized for expression of the antibody of the invention or the corresponding immunoglobulin chains in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. Suitable source cells for the DNA sequences and host cells for immunoglobulin expression and secretion can be obtained from a number of sources, such as the American Type Culture Collection ("Catalogue of Cell Lines and Hybridomas," Fifth edition (1985) Rockville, Maryland, U.S.A., which is incorporated herein by reference). Furthermore, transgenic animals, preferably mammals, comprising cells of the invention may be used for the large scale production of the antibody of the invention.

Thus, in a further embodiment, the present invention relates to a method for the production of an antibody or a binding fragment or immunoglobulin chain(s) thereof, said method comprising
(a) culturing a cell as described above; and
(b) isolating said antibody or binding fragment or immunoglobulin chain(s) thereof from the culture.

The transformed hosts can be grown in fermentors and cultured according to techniques known in the art to achieve optimal cell growth. Once expressed, the whole antibodies, their dimers, individual light and heavy chains, or other immunoglobulin forms of the present invention, can be purified according to standard procedures of the art, including ammonium sulfate precipitation, affinity columns, column chromatography, gel electrophoresis and the like; see, Scopes, "Protein Purification", Springer Verlag, N.Y. (1982). The antibody or its corresponding immunoglobulin chain(s) of the invention can then be isolated from the growth medium, cellular lysates, or cellular membrane fractions. The isolation and purification of the, e.g., recombinantly expressed antibodies or immunoglobulin chains of the invention may be by any conventional means such as, for example, preparative chromatographic separations and immunological separations such as those involving the use of monoclonal or polyclonal antibodies directed, e.g., against the constant region of the antibody of the invention. It will be apparent to those skilled in the art that the antibodies of the invention can be further coupled to other moieties for, e.g., drug targeting and imaging applications. Such coupling may be conducted chemically after expression of the antibody to site of attachment or the coupling product may be engineered into the antibody of the invention at the DNA level. The DNAs are then expressed in a suitable host system, and the expressed proteins are collected and renatured, if necessary.

Substantially pure immunoglobulins of at least about 90 to 95% homogeneity are preferred, and 98 to 99% or more homogeneity most preferred, for pharmaceutical uses. Once purified, partially or to homogeneity as desired, the antibodies may then be used therapeutically (including extracorporally) or in developing and performing assay procedures.

The present invention also involves a method for producing cells capable of expressing an antibody of the invention or its corresponding immunoglobulin chain(s) comprising genetically engineering cells with the polynucleotide or with the vector of the invention. The cells obtainable by the method of the invention can be used, for example, to test the interaction of the antibody of the invention with its antigen.

As mentioned before, the immunoglobulin or its encoding cDNAs may be further modified. Thus, in a further embodiment the method of the present invention comprises any one of the step(s) of producing a chimeric antibody, humanized antibody, single-chain antibody, Fab-fragment, bi-specific antibody, fusion antibody, labeled antibody or an analog of any one of those. Corresponding methods are known to the person skilled in the art and are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to the same epitope as that of any one of the antibodies described herein (Schier, Human Antibodies Hybridomas 7 (1996), 97-105; Malmborg, J. Immunol. Methods 183 (1995), 7-13). The production of chimeric antibodies is described, for example, in international application WO89/09622. Methods for the production of humanized antibodies are described in, e.g., European application EP-A1 0 239 400 and international application WO90/07861. A further source of antibodies to be utilized in accordance with the present invention are so-called xenogeneic antibodies. The general principle for the production of xenogeneic antibodies such as human antibodies in mice is described in, e.g., international applications WO91/10741, WO94/02602, WO96/34096 and WO 96/33735. As discussed above, the antibody of the invention may exist in a variety of forms besides complete antibodies; including, for example, Fv, Fab and F(ab)₂, as well as in single chains; see e.g. international application WO88/09344. Furthermore, diabodies and V-like domain binding molecules are well-known to the person skilled in the art; see, e.g. US patent No. 7,166,697.

The antibodies of the present invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Modifications of the antibody of the invention include chemical and/or enzymatic derivatizations at one or more constituent amino acids, including side chain modifications, backbone modifications, and N- and C-terminal modifications including acetylation, hydroxylation, methylation, amidation, and the attachment or removal of carbohydrate or lipid moieties, cofactors, and the like. Likewise, the present invention encompasses the production of chimeric proteins which comprise the described antibody or some fragment thereof at the amino terminus fused to heterologous molecule such as an immunostimulatory ligand at the carboxyl terminus; see, e.g., international application WO00/30680 for corresponding technical details.

Additionally, the present invention encompasses small peptides including those containing a binding molecule as described above, for example containing the CDR3 region of the variable region of any one of the mentioned antibodies, in particular CDR3 of the heavy chain since it has frequently been observed that heavy chain CDR3 (HCDR3) is the region having a greater degree of variability and a predominant participation in antigen-antibody interaction. Such peptides may easily be synthesized or produced by recombinant means to produce a binding agent useful according to the invention. Such methods are well known to those of ordinary skill in the art. Peptides can be synthesized for example, using automated peptide synthesizers which are commercially available. The peptides can be produced by recombinant techniques by incorporating the DNA expressing the peptide into an expression vector and transforming cells with the expression vector to produce the peptide.

Hence, the present invention relates to any binding molecule, antibody or binding fragment obtainable in accordance with above described means and display the mentioned properties.

In a further embodiment of the present invention, the binding molecule, antibody, immunoglobulin chain or a binding fragment thereof or the antigen is detectably labeled. Labeling agents can be coupled either directly or indirectly to the antibodies or antigens of the invention. One example of indirect coupling is by use of a spacer moiety. Furthermore, the antibodies of the present invention can comprise a further domain, said domain being linked by covalent or non-covalent bonds. The linkage can be based on genetic fusion according to the methods known in the art and described above or can be performed by, e.g., chemical cross-linking as described in, e.g., international application WO94/04686. The additional domain present in the fusion protein comprising the antibody of the invention may preferably be linked by a flexible linker, advantageously a polypeptide linker, wherein said polypeptide linker comprises plural, hydrophilic, peptide-bonded amino acids of a length sufficient to span the distance between the C-terminal end of said further domain and the N-terminal end of the antibody of the invention or vice versa. The therapeutically or diagnostically active agent can be coupled to the antibody of the invention or an antigen-binding fragment thereof by various means. This includes, for example, single-chain fusion proteins comprising the variable regions of the antibody of the invention coupled by covalent methods, such as peptide linkages, to the therapeutically or diagnostically active agent. Further examples include molecules which comprise at least an antigen-binding fragment coupled to additional molecules covalently or non-covalently include those in the following non-limiting illustrative list. Traunecker, Int. J. Cancer Surp. SuDP 7 (1992), 51-52, describe the bispecific reagent janusin in which the Fv region directed to CD3 is coupled to soluble CD4 or to other ligands such as OVCA and IL-7. Similarly, the variable regions of the antibody of the invention can be constructed into Fv molecules and coupled to alternative ligands such as those illustrated in the cited article. Higgins, J. Infect Disease 166 (1992), 198-202, described a hetero-conjugate antibody composed of OKT3 cross-linked to an antibody directed to a specific sequence in the V3 region of GP120. Such hetero-conjugate antibodies can also be constructed using at least the variable regions contained in the antibody of the invention methods. Additional examples of specific antibodies include those described by Fanger, Cancer Treat. Res. 68 (1993), 181-194 and by Fanger, Crit. Rev. Immunol. 12 (1992), 101-124. Conjugates that are immunotoxins including conventional antibodies have been widely described in the art. The toxins may be coupled to the antibodies by conventional coupling techniques or immunotoxins containing protein toxin portions can be produced as fusion proteins. The antibodies of the present invention can be used in a corresponding way to obtain such immunotoxins. Illustrative of such immunotoxins are those described by Byers, Seminars Cell. Biol. 2 (1991), 59-70 and by Fanger, Immunol. Today 12 (1991), 51-54.

The above described fusion protein may further comprise a cleavable linker or cleavage site for proteinases. These spacer moieties, in turn, can be either insoluble or soluble (Diener et al., Science 231 (1986), 148) and can be selected to enable drug release from the antibody at the target site. Examples of therapeutic agents which can be coupled to the antibodies of the present invention for immunotherapy are drugs, radioisotopes, lectins, and toxins. The drugs with which can be conjugated to the antibodies and antigens of the present invention include compounds which are classically referred to as drugs such as mitomycin C, daunorubicin, and vinblastine. In using radioisotopically conjugated antibodies or antigens of the invention for, e.g., immunotherapy, certain isotopes may be more preferable than others depending on such factors as leukocyte distribution as well as stability and emission. Depending on the autoimmune response, some emitters may be preferable to others. In general, α and ß particle emitting radioisotopes are preferred in immunotherapy. Preferred are short range, high energy a emitters such as ²¹²Bi. Examples of radioisotopes which can be bound to the antibodies or antigens of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹Pd and ¹⁸⁸Re. Most preferably, the radiolabel is ⁶⁴Cu. Other therapeutic agents which can be coupled to the antibody or antigen of the invention, as well as ex vivo and in vivo therapeutic protocols, are known, or can be easily ascertained, by those of ordinary skill in the art. Wherever appropriate the person skilled in the art may use a polynucleotide of the invention encoding any one of the above described antibodies, antigens or the corresponding vectors instead of the proteinaeous material itself.

Hence, the biological activity of the binding molecules, e.g. antibodies identified here suggests that they have sufficient affinity to make them potential candidates for drug localization to cells or tissue displaying the appropriate surface structures of for example Abeta deposits. This targeting and binding to Abeta deposits could be useful for the delivery of therapeutically or diagnostically active agents and gene therapy/gene delivery. Molecules/particles with an antibody of the invention therefore could have diagnostic and therapeutic use. Thus, the antibody of the present invention can be labeled (e.g., fluorescent, radioactive, enzyme, nuclear magnetic, heavy metal) and used to detect specific targets in vivo or in vitro including "immunochemistry" like assays in vitro. In vivo they could be used in a manner similar to nuclear medicine imaging techniques to detect tissues, cells, or other material contaminated with Abeta peptide and deposits thereof. Targeting Abeta peptides and amyloid plaques with diagnostic imaging probes detectable by MRI or PET would provide a biological marker for a more definitive premortem diagnosis of AD and a means for monitoring the efficacy of beta-amyloid lowering therapies.

Thus, in a further embodiment the present invention relates to the use of a binding molecule or an antibody of the present invention or binding fragment thereof for the preparation of a composition for in vivo detection of or targeting a therapeutic and/or diagnostic agent to Abeta deposits in the brain, detecting, suppressing formation of or reducing pathological Abeta aggregates or conformations in a subject, for improving cognition or slowing or reversing cognitive decline associated with diseases, or for extra-corporal extraction of pathological compounds or their precursors from body fluids.

In a further embodiment the present invention relates to an extracorporal or implanted medical device such as an apharesis device containing the aforementioned binding molecule, antibody or binding fragment of the present invention or chemical derivatives thereof immobilized to a matrix or confided to a separate compartment to allow removal of the Abeta peptide from blood or cerebrospinal fluid (CSF) by affinity capture or plasmapharesis. Alternatively the antibody or binding fragment of the present invention or chemical derivatives thereof may be administered directly to the blood or CSF and sequestered in a subsequent step by affinity capture from the blood or CSF, whereby Abeta peptide is sequestered together with the aforementioned binding molecule. Hence, the present invention also relates to a method of treating or preventing the onset or progression of Alzheimer's disease or amyloidosis in a subject comprising removing blood or CSF from the body of the subject, subjecting the blood and CSF, respectively, to an apharesis device of the present invention, and returning the blood and CSF, respectively, so obtained to the subject.

Moreover, the present invention relates to compositions comprising the aforementioned binding molecule, antibody or binding fragment of the present invention or chemical derivatives thereof, or the polynucleotide, vector or cell of the invention. The composition of the present invention may further comprise a pharmaceutically acceptable carrier. The term "chemical derivative" describes a molecule that contains additional chemical moieties that are not normally a part of the base molecule. Such moieties may improve the solubility, half-life, absorption, etc. of the base molecule. Alternatively the moieties may attenuate undesirable side effects of the base molecule or decrease the toxicity of the base molecule. Furthermore, the pharmaceutical composition of the present invention may comprise further agents such as interleukins or interferons depending on the intended use of the pharmaceutical composition. For example, for use in the treatment of Alzheimer's disease the additional agent may selected from the group consisting of small organic molecules, anti-Abeta antibodies, and combinations thereof. Hence, in a particular preferred embodiment the present invention relates to the use of the binding molecule, antibody or binding fragment of the present invention or of a binding molecule having substantially the same binding specificities of any one thereof, the polynucleotide, the vector or the cell of the present invention for the preparation of a pharmaceutical or diagnostic composition for treating or preventing the progression of Alzheimer's disease or amyloidosis; for the amelioration of symptoms associated with Alzheimer's disease or amyloidosis; for diagnosing or screening a subject for the presence of Alzheimer's disease or amyloidosis for determining a subject's risk for developing Alzheimer's disease or amyloidosis.

Hence, in one embodiment the present invention relates to a method of treating a neurological disorder or amyloidosis, including peripheral amyloidosis such as inclusion body myositis, characterized by abnormal accumulation and/or deposition of Abeta, which method comprises administering to a subject in need thereof a therapeutically effective amount of any one of the afore-described binding molecules, antibodies, polynucleotides, vectors or cells of the instant invention. The term "neurological disorder" includes but is not limited to Alzheimer's Disease, mild cognitive impairment, progressive supranuclear palsy, cortico-basal degeneration, agyrophilic grain disease, fronto-temporal dementia, fronto-temporal dementia with Parkinsonism, Lewy-body disease, Parkinson's disease, Pick's disease, Binswanger's disease, cerebral amyloid angiopathy, Down's syndrome, hereditary cerebral hemorrhage with amyloidosis Dutch type and Icelandic type, multi-infarct dementia, Huntington's Disease, Creutzfeldt-Jakob Disease, AIDS dementia complex, depression, anxiety disorder, phobia, Bell's Palsy, epilepsy, encephalitis, multiple sclerosis; neuromuscular disorders, neurooncological disorders, brain tumors, neurovascular disorders including stroke, neuroimmunological disorders, neurootological disease, neurotrauma including spinal cord injury, pain including neuropathic pain, pediatric neurological and neuropsychiatric disorders, sleep disorders, Tourette syndrome, mild cognitive impairment, vascular dementia, multi-infarct dementia, cystic fibrosis, Gaucher's disease other movement disorders and disease of the central nervous system (CNS) in general. Unless stated otherwise, the terms neurodegenerative, neurological or neuropsychiatric are used interchangeably herein.

From the foregoing, it is evident that the present invention encompasses any use of a binding molecule comprising at least one CDR of the above described antibody, in particular for diagnosing and/or treatment of a disorder related to Alzheimer's disease and Abeta deposition, respectively. Preferably, said binding molecule is an antibody of the present invention or an immunoglobulin chain thereof. In addition, the present invention relates to anti-idiotypic antibodies of any one of the mentioned antibodies described hereinbefore. These are antibodies or other binding molecules which bind to the unique antigenic peptide sequence located on an antibody's variable region near the antigen binding site.

In another embodiment the present invention relates to a diagnostic composition comprising any one of the above described binding molecules, antibodies, antigen-binding fragments, polynucleotides, vectors or cells of the invention and optionally suitable means for detection such as reagents conventionally used in immuno or nucleic acid based diagnostic methods. The antibodies of the invention are, for example, suited for use in immunoassays in which they can be utilized in liquid phase or bound to a solid phase carrier. Examples of immunoassays which can utilize the antibody of the invention are competitive and non-competitive immunoassays in either a direct or indirect format. Examples of such immunoassays are the radioimmunoassay (RIA), the sandwich (immunometric assay), flow cytometry and the Western blot assay. The antibodies of the invention can be bound to many different carriers and used to isolate cells specifically bound thereto. Examples of well known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds; see also the embodiments discussed hereinabove.

By a further embodiment, the binding molecules, in particular antibodies of the present invention may also be used in a method for the diagnosis of a disorder in an individual by obtaining a body fluid sample from the tested individual which may be a blood sample, a lymph sample or any other body fluid sample and contacting the body fluid sample with an antibody of the instant invention under conditions enabling the formation of antibody-antigen complexes. The level of such complexes is then determined by methods known in the art, a level significantly higher than that formed in a control sample indicating the disease in the tested individual. Thus, the present invention relates to an in vitro immunoassay comprising the antibody of the invention.

Furthermore, the present invention relates to in vivo imaging techniques employing any one of the binding molecules of the present invention. For example, the medical imaging technique Positron emission tomography (PET) which produces a three-dimensional image of body parts is based on the detection of radiation from the emission of positrons. Typically, a biomolecule is radioactively labeled, e.g. it incorporates a radioactive tracer isotope. Upon administration of the labeled biomolecule to the subject, typically by injection into the blood circulation, the radioactively labeled biomolecule becomes concentrated in tissues of interest. The subject is then placed in the imaging scanner, which detects the emission of positrons. In one embodiment, a labeled, preferably ⁶⁴Cu labeled binding molecule such as an antibody is administered to a subject and detection of the binding molecule and thus Abeta peptide is performed by placing the subject in an imaging scanner and detecting the emission of positrons, thereby indicating a neurological disorder if emission is detected. The present invention thus encompasses a method for PET imagining, comprising the step of administering a ⁶⁴Cu-labelled or equivalent labeled binding molecule of the present invention to a subj ect.

In this context, the present invention also relates to means specifically designed for this purpose. For example, an antibody-based array may be used, which is for example loaded with antibodies or equivalent antigen-binding molecules of the present invention which specifically recognize Abeta peptides. Design of microarray immunoassays is summarized in Kusnezow et al., Mol. Cell Proteomics 5 (2006), 1681-1696. Accordingly, the present invention also relates to microarrays loaded with binding molecules in accordance with the present invention.

The present invention also provides a pharmaceutical and diagnostic, respectively, pack or kit comprising one or more containers filled with one or more of the above described ingredients, i.e. binding molecule, antibody or binding fragment thereof, polynucleotide, vector or cell of the present invention. Associated with such container(s) can be a notice in the form prescribed by a governmental agency regulating the manufacture, use or sale of pharmaceuticals or biological products, which notice reflects approval by the agency of manufacture, use or sale for human administration. In addition or alternatively the kit comprises reagents and/or instructions for use in appropriate diagnostic assays. The composition, i.e. kit of the present invention is of course particularly suitable for the diagnosis, prevention and treatment of a disorder which is accompanied with the presence of a disorder-associated protein as defined above, especially amyloidosis, and in particular applicable for the treatment of Alzheimer's disease (AD); see also supra.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e. arresting its development; or (c) relieving the disease, i.e. causing regression of the disease.

Furthermore, the term "subject" or "patient" refers to a mammal, preferably a human, in need of treatment for a condition, disorder or disease.

The pharmaceutical compositions of the present invention can be formulated according to methods well known in the art; see for example Remington: The Science and Practice of Pharmacy (2000) by the University of Sciences in Philadelphia, ISBN 0-683-306472. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. Aerosol formulations such as nasal spray formulations include purified aqueous or other solutions of the active agent with preservative agents and isotonic agents. Such formulations are preferably adjusted to a pH and isotonic state compatible with the nasal mucous membranes. Formulations for rectal or vaginal administration may be presented as a suppository with a suitable carrier.

Furthermore, whereas the present invention includes the now standard (though fortunately infrequent) procedure of drilling a small hole in the skull to administer a drug of the present invention, in a preferred aspect, the binding molecule, especially antibody or antibody based drug of the present invention can cross the blood-brain barrier, which allows for intravenous or oral administration.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as dopamine or psychopharmacologic drugs, depending on the intended use of the pharmaceutical composition. Furthermore, the pharmaceutical composition may also be formulated as a vaccine, for example, if the pharmaceutical composition of the invention comprises an anti-Abeta antibody for passive immunization.

In addition, co-administration or sequential administration of other agents may be desirable. A therapeutically effective dose or amount refers to that amount of the active ingredient sufficient to ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

Preferably, the therapeutic agent in the composition is present in an amount sufficient to restore normal behavior and/or cognitive properties in case of Alzheimer's disease.

The pharmaceutical compositions in accordance with the present invention can preferably be used for the treatment of neurological disorders including but not limited to Alzheimer's disease, aphasia, Bell's Palsy, Creutzfeldt-Jakob disease, epilepsy, encephalitis, Huntington's disease, neuromuscular disorders, neuro-oncology, neuro-immunology, neuro-otology pain, pediatric neurology, phobia sleep disorders, Tourette Syndrome, Parkinson's disease, other movement disorders and disease of the central nervous system (CNS) in general.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### EXAMPLES

The examples which follow further illustrate the invention, but should not be construed to limit the scope of the invention in any way. Detailed descriptions of conventional methods, such as those employed herein can be found in the cited literature; see also "The Merck Manual of Diagnosis and Therapy" Seventeenth Ed. edited by Beers and Berkow (Merck & Co., Inc. 2003).

The practice of the present invention will employ, unless otherwise indicated, conventional techniques of cell biology, cell culture, molecular biology, transgenic biology, microbiology, recombinant DNA, and immunology, which are within the skill of the art. For further elaboration of general techniques useful in the practice of this invention, the practitioner can refer to standard textbooks and reviews in cell biology and tissue culture; see also the references cited in the examples. General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Protein Methods (Bollag et al., John Wiley & Sons 1996); Non-viral Vectors for Gene Therapy (Wagner et al. eds., Academic Press 1999); Viral Vectors (Kaplitt & Loewy eds., Academic Press 1995); Immunology Methods Manual (Lefkovits ed., Academic Press 1997); and Cell and Tissue Culture: Laboratory Procedures in Biotechnology (Doyle & Griffiths, John Wiley & Sons 1998). Reagents, cloning vectors and kits for genetic manipulation referred to in this disclosure are available from commercial vendors such as BioRad, Stratagene, Invitrogen, Sigma-Aldrich, and ClonTech.General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11 (2001), 98-107.

### Supplementary Methods

Experimental procedures concerning transgenic mice expressing the familial Alzheimer's disease-causing Swedish mutation of amyloid precursor protein (SwAPP), passive immunization protocols and Abeta ELISA are described by Mohajeri et al, in J. Bio. Chem. 277 (2002), 33012-33017. Furthermore, an in vitro test system for the efficacy of microglia cells to take up fibrillar Abeta and Abeta/antibody microaggregates, respectively, as well as passive immunization of transgenic SwAPP mice are described in Mohajeri et al., Neurodegenerative Dis. 1 (2004), 160-167. The Mohajeri et al. references are incorporated herein in their entirety for their experimental protocols and references cited therein for use of the assessment of the bioactivity of antibodies against Abeta peptide in vitro and in vivo.

### ELISA

96 well half area Microplates (Coming) were coated with synthetic Abeta-peptide preparations at a standard concentration of 1 µg/ml in coating buffer (15mM Na₂CO₃, 35 mM NaHCO₃, pH 9.42) overnight at 4°C. Plates were washed and non-specific binding sites were blocked for 1h at RT with PBS containing 2% BSA (Sigma, Buchs, Switzerland). Antibody NI-103 was added to ELISA plates at the indicated concentrations and was incubated for 2 h at room temperature. Binding of NI-103 was determined using horse radish peroxidase (HRP)-conjugated anti-mouse IgG polyclonal antibodies (Jackson ImmunoResearch Europe Ltd., Cambridgeshire, UK) followed by measurement of HRP activity in a standard colorimetric assay.

### Preparation of Abeta fibrils

Abeta-peptide was purchased from Bachem (Bubendorf, Switzerland). Lyophilized peptide was reconstituted in TFA and resuspended in PBS immediately prior to its use as monomeric Abeta in the assays. Abeta fibrils were prepared by incubation of monomeric Abeta-peptide 1-42 at a concentration of 100µg/ml in PBS at 37°C for 24h.

### Immunofluorescence staining of living cells

HEK 293 cells were transiently transfected with a vector that expresses human wild type APP fused at the intracellular C-terminus to the yellow fluorescent protein variant Citrine. 24 hours after transfection the cells were incubated with human recombinant antibodies or control antibodies at 4°C for 30 minutes. After a washing step the cell were fixed and surface-bound antibody was detected using Cy-3-labeled secondary antibodies to human or mouse IgG (Jackson ImmunoResearch). Analysis of fluorescence was performed on a confocal microscope (Leica).

### Example 1: NI-103 antibody binds to beta-amyloid protein in brain amyloid plaques but not the physiological amyloid precursor protein

NI-103 was tested for binding to brain beta-amyloid plaques (Fig. 2). Brain sections obtained from a patient with neuropathologically confirmed Alzheimer's disease were stained with NI-103 antibody at 10 nM concentration. Antibody binding to beta-amyloid plaques was detected with a fluorescently labelled secondary antibody specific for mouse IgG. As a control, staining was performed with the secondary antibody only.

Cross-reactivity of recombinant human NI-103 antibody against cellular full-length APP or with any of its physiological derivatives was determined by cell binding assays (Fig. 3). Live HEK 293 cells stably expressing human APP fused to Citrin as a marker were incubated for 30 min at 4°C, to prevent internalization, with the recombinant human NI-103 antibody or the control antibody 6E10 against N-terminal linear Abeta sequence. Citrin-positive signals indicate APP-expressing cells. In contrast to the control antibody (6E10) that binds to cell-surface APP in all cells expressing the fusion construct, no binding of recombinant human NI-103 antibody to full-length APP is detected. These data demonstrate absent cross-reactivity of NI-103 to physiological, cellular APP.

### Example 2: NI-103 antibody binds to synthetic preparations of monomeric Abetal-40 and Abetal-42 and fibrillar Abetal-42 with substantially identical affinity

The binding of NI-103 to synthetic Abetal-40 or Abetal-42 monomers or Abetal-42 fibril preparations was determined by ELISA (Fig. 4). Synthetic Abeta preparations coated onto ELISA plates at equal coating densities were incubated with NI-103 at the indicated concentrations. High affinity binding of NI-103 was observed with substantially identical sub 0.1 nM EC50 for all Abeta species assayed (EC50 for monomeric Abetal-40: 0.040nM; monomeric Abeta1-42: 0.061nM; fibrillar Abeta1-42: 0.070nM).

### Example 3: NI-103 antibody binds to a C-terminal epitope present in Abetal-40 and Abetal-42

To determine the Abeta epitope that is recognized by NI-103, binding of NI-103 to different species and fragments of the amyloid beta peptide was analyzed by ELISA. Comparable signal was measured for the full length Abetal-40 and Abetal-42 peptides indication high affinity binding of NI-103 to both peptide species (Fig. 5a, b). No binding of NI-103 was observed to Abeta1-11, Abetal-28 Abeta17-28 and Abetal-38, suggesting specific binding to a C-terminal epitope (Fig. 5a-c). The absence of binding to a peptide consisting of amino acids 22-35 and 33-42 of Abeta, but intermediate binding to an Abeta29-40 fragment suggests a C-terminal epitope contained within amino acids 29-40 (Fig. 5a-c). In a competition ELISA, binding of 0.5 nM NI-103 to Abetal-42 was completely blocked by pre-incubation of the antibody with 1 µM concentrations of Abetal-40, Abetal-42 and Abeta29-40, but not Abetal-38 or Abeta33-42, suggesting that NI-103 recognizes a C-terminal epitope and requires a strech of amino acids contained within residues 29-40 for binding (Fig. 6).

### Example 4: Systemic treatment with NI-103 antibody improves cognitive behavior and rescues the number of immature neurons in a transgenic mouse model of Alzheimer's disease

Neurogenesis occurs in the adult mammalian brain in two defined and specific areas: the subventricular zone (SVZ) of the lateral ventricle wall and the subgranular zone (SGZ) in the dentate gyrus of the hippocampus. The hippocampus is one of the most affected brain regions areas by Alzheimer's disease (AD), pathologically defined by atrophy, amyloid plaques and neurofibrillary tangles. The effect of amyloid pathology on neurogenesis is still controversial. Both, decreased as well as increased neurogenesis have been reported by several groups in different transgenic mouse models of AD. The effects of amyloidosis and therapeutic intervention with NI-103 on neurogenesis were studied in APPswe/PS1 double transgenic mice (Hsiao et al., Science 274 (1996), 99-102; Duff et al., Nature 383 (1996) 710-713). To that end, 8 months old APPswe/PS1 were injected i.v. with 5 mg/kg of either NI-103 or PBS in 5 day intervals. Wild type littermates were treated accordingly with PBS. All animal received Bromodeoxyuridine (BrdU) injections (i.p., twice daily, 50 mg/kg) starting 2 weeks before sacrifice. Spontenous alternation behavior and exploratory activity analysis was evaluated by behavioral testing in the Y-maze. Animals were sacrificed at 11 month or age. PFA-fixed brains were processed for free floating immunohistochemical analysis (30 µm sections, 8 series) and stained for BrdU/NeuN (mature neurons) and DCX (immature neurons) and counted in 3 coronal sections per animal.

At 11 month of age, APPswe/PS1 mice were significantly impaired in the Y-maze compared to their wild-type littermates (Fig. 7; p=0.01). Passive immunization with NI-103 was associated with significant improvement in working memory, as suggested by the increased frequency of spontaneous alternation (Fig. 7; p=0.01). The histological analysis revealed an elevated number of proliferating BrdU+ cells in control-treated or NI-103 treated APPswe/PS1 transgenic mice compared to their wild-type littermates (p<0.05). A similar trend was observed for the number of BrdU/NeuN double positive neurons. In contrast, APP transgenic mice had reduced numbers of immature DCX+ neurons in the dentate gyrus compared to their wild-type littermates (p<0.05). Systemic treatment with NI-103 for 3 month restored the numbers of DCX+ neurons to almost wt levels, suggesting a protective effect of NI-103 treatment on immature neurons (Fig. 8).

### Conclusion

As demonstrated in the above experiments performed in accordance with the present invention a monoclonal antibody could be isolated and secured which has unique immunological binding characteristics such as binding monomeric and fibrillar Abeta42 peptide as well as binding to a C-terminal epitope present in Abeta40 and Abeta42 with substantially identical affinity but without crossreactivity to cellular full length APP or APP derivatives. This makes the antibody and equivalent binding molecules particularly suitable for diagnostic and therapeutic applications, since the antibody discriminates the physiologically functional form of APP, and thereby minimizing the risk of side effects. Furthermore, as demonstrated in Example 1, the antibody of the present invention is capable of binding to amyloid plaques in the brain. In addition, Example 4 demonstrates the therapeutic activity of the antibody by improving cognitive behavior and preventing brain damage in transgenic mice displaying an Alzheimer's disease related phenotype. Thus, antibodies and equivalent binding molecules are provided that specifically recognize Abeta peptides, which the antibody is supposed to bind in order to diminish Abeta-related toxicity or to reduce Abeta concentration or to promote its degradation, by means of, for example, inducing FcR mediated uptake of beta amyloid by macrophages or microglial cells. As further demonstrated in the examples such antibodies are therapeutically effective and are capable of both suspending as well as preventing deleterious effects Abeta on cognitive behavior and immature neurons.

## Claims

1. An anti-amyloid beta peptide (Abeta) monoclonal antibody, which antibody is capable of
(a) recognizing the C-terminus of both the Abeta42 and Abeta40 peptide;
(b) binding monomeric and aggregated forms of Abeta;
(c) not substantially recognizing cellular full length amyloid precursor protein (APP) **characterized by** having a preferential binding affinity to Abeta over APP by a factor of at least two;
(d) recognizing an epitope contained within the amino acid sequence GAIIGLMVGGVV (SEQ ID NO: 1); and wherein
(e) the antibody comprises the complementarity determining regions (CDRs) comprising the amino acid sequences as set forth in SEQ ID NOs: 6 to 11 and/or wherein one or more of the CDRs comprise one or more amino acid substitutions.

2. The antibody of claim 1, which is capable of mediating the uptake of fibrillar Abeta by microglia and/or binding to amyloid plaques in the brain.

3. A monoclonal antibody or antigen-binding fragment thereof which demonstrates the immunological binding characteristics of the antibody of claim 1 or 2.

4. The antibody of any one of claims 1 to 3, which is a humanized, or a chimeric human-murine antibody and/or is selected from the group consisting of a single chain antibody (scFv), an F(ab') fragment, an F(ab) fragment, and an F(ab')2 fragment.

5. The antibody of any one of claims 1 to 4, comprising in its epitope binding domain at least one complementarity determining region (CDR) of the V_{H} and/or V_{L} chain of the variable region comprising the amino acid sequence (V_{H}) (SEQ ID NO: 2) and (V_{L}) (SEQ ID NO: 4) depicted in Figure 1.

6. A polynucleotide encoding at least the binding domain or variable region of an immunoglobulin chain of the antibody of any one of claims 1 to 5.

7. A vector comprising the polynucleotide of claim 6.

8. A host cell comprising a polynucleotide of claim 6 or a vector of claim 7.

9. A method for preparing an antibody or immunoglobulin chain(s) thereof, said method comprising
(a) culturing the cell of claim 8; and
(b) isolating said antibody or immunoglobulin chain(s) thereof from the culture.

10. An antibody or immunoglobulin chain(s) thereof encoded by a polynucleotide of claim 6 or obtained by the method of claim 9.

11. The antibody of any one of claims 1 to 5 or 10, which is
(i) detectably labelled, preferably wherein the detectable label is selected from the group consisting of an enzyme, a radioisotope, a fluorophore and a heavy metal; and/or
(ii) attached to a drug.

12. A composition comprising the antibody of any one of claims 1 to 5, 10 or 11, the polynucleotide of claim 6, the vector of claim 7 or the cell of claim 8, preferably which is
(i) a pharmaceutical composition and further comprises a pharmaceutically acceptable carrier, preferably further comprising an additional agent for the use in treating Alzheimer's disease or amyloidosis, selected from the group consisting of small organic molecules, anti-Abeta antibodies, and combinations thereof; or
(ii) a diagnostic composition, optionally comprising reagents conventionally used in immuno or nucleic acid based diagnostic methods.

13. The pharmaceutical composition of claim 12, which is designed to be administered intravenously, intramuscularly, subcutaneously, intraperitoneally, intranasally, or as an aerosol.

14. An apharesis device comprising the antibody of any one of claims 1 to 5, 10 or 11, preferably is a plasmapheresis device.

15. A kit for the use in diagnosis of Alzheimer's disease or amyloidosis, said kit comprising the antibody of any one of claims 1 to 5, 10 or 11, the polynucleotide of claim 6, the vector of claim 7 or the cell of claim 8, with reagents and/or instructions for use.

16. An antibody of any one of claims 1 to 5, 10 or 11, an Abeta binding fragment thereof or an Abeta binding molecule having substantially the same binding specificities of any one thereof, the polynucleotide of claim 6, the vector of claim 7 or the cell of claim 8 for the preparation of a pharmaceutical or diagnostic composition for use in treating or preventing the progression of Alzheimer's disease or amyloidosis; for the amelioration of symptoms associated with Alzheimer's disease or amyloidosis; for diagnosing or screening a subject for the presence of Alzheimer's disease or amyloidosis for determining a subject's risk for developing Alzheimer's disease or amyloidosis.

17. An Abeta binding molecule comprising at least one CDR of an antibody of any one of claims 1 to 5, 10 or 11 for use in in vivo detection or imaging of or targeting a therapeutic and/or diagnostic agent to Abeta in the brain, preferably wherein said in vivo imaging comprises positron emission tomography (PET).


SEQUENCE LISTING

<110>  University of Zurich

<120>  Monoclonal amyloid beta (Abeta)-specific antibody and uses
       thereof

<130>  NE30A12/P-WO

<150>  60/993,749
<151>  2007-09-13

<160>  11

<170>  PatentIn version 3.5

<210>  1
<211>  12
<212>  PRT
<213>  Artificial

<220>
<223>  Peptide

<220>
<221>  PEPTIDE
<222>  (1)..(12)
<223>  Epitope of anti-amyloid beta peptide (Abeta) recognized by
       antibody NI-103

<400>  1

Gly Ala Ile Ile Gly Leu Met Val Gly Gly Val Val
1               5                   10

<210>  2
<211>  366
<212>  DNA
<213>  Mus musculus

<220>
<221>  CDS
<222>  (1)..(366)
<223>  NI-103 variable heavy (Vh) chain sequence

<400>  2
gag gtc cag ctg cat cag tct gga cct gaa ctg gtg aag cct ggg gct       48
Glu Val Gln Leu His Gln Ser Gly Pro Glu Leu Val Lys Pro Gly Ala
1               5                   10                  15

tca gtg aag ata tcc tgc aag act tct gga tac aca ttc act gaa tac       96
Ser Val Lys Ile Ser Cys Lys Thr Ser Gly Tyr Thr Phe Thr Glu Tyr
            20                  25                  30

acc atg cac tgg gtg aaa cag agc cat gga aag agc ctt gag tgg att      144
Thr Met His Trp Val Lys Gln Ser His Gly Lys Ser Leu Glu Trp Ile
        35                  40                  45

gga ggt gtt aat cct tac aat gat aat act tct tac atc cgg aag tta      192
Gly Gly Val Asn Pro Tyr Asn Asp Asn Thr Ser Tyr Ile Arg Lys Leu
    50                  55                  60

cag ggc aag gtc aca ttg act gta gac agg tcc tcc agc aca gcc tac     240
Gln Gly Lys Val Thr Leu Thr Val Asp Arg Ser Ser Ser Thr Ala Tyr
65                  70                  75                  80

atg gag ctc cgc agc ctg aca tct gag gat tct gca gtc tat ttc tgt     288
Met Glu Leu Arg Ser Leu Thr Ser Glu Asp Ser Ala Val Tyr Phe Cys
                85                  90                  95

gca aga tac ggg gga tta cgc cct tat tac ttt cct atg gac ttc tgg     336
Ala Arg Tyr Gly Gly Leu Arg Pro Tyr Tyr Phe Pro Met Asp Phe Trp
            100                 105                 110

ggt caa gga acc tca gtc acc gtc tcc tca                             366
Gly Gln Gly Thr Ser Val Thr Val Ser Ser
        115                 120

<210>  3
<211>  122
<212>  PRT
<213>  Mus musculus

<400>  3

Glu Val Gln Leu His Gln Ser Gly Pro Glu Leu Val Lys Pro Gly Ala
1               5                   10                  15

Ser Val Lys Ile Ser Cys Lys Thr Ser Gly Tyr Thr Phe Thr Glu Tyr
            20                  25                  30

Thr Met His Trp Val Lys Gln Ser His Gly Lys Ser Leu Glu Trp Ile
        35                  40                  45

Gly Gly Val Asn Pro Tyr Asn Asp Asn Thr Ser Tyr Ile Arg Lys Leu
    50                  55                  60

Gln Gly Lys Val Thr Leu Thr Val Asp Arg Ser Ser Ser Thr Ala Tyr
65                  70                  75                  80

Met Glu Leu Arg Ser Leu Thr Ser Glu Asp Ser Ala Val Tyr Phe Cys
                85                  90                  95

Ala Arg Tyr Gly Gly Leu Arg Pro Tyr Tyr Phe Pro Met Asp Phe Trp
            100                 105                 110

Gly Gln Gly Thr Ser Val Thr Val Ser Ser
        115                 120

<210>  4
<211>  321
<212>  DNA
<213>  Mus musculus

<220>
<221>  CDS
<222>  (1)..(321)

<223>  NI-103 variable light (Vl) chain sequence

<400>  4
caa gtt gtt ctc tcc cag tct cca gca atc ctg tct gca tct cca ggg      48
Gln Val Val Leu Ser Gln Ser Pro Ala Ile Leu Ser Ala Ser Pro Gly
1               5                   10                  15

gag agg gtc aca ctg act tgc agg gcc agc tca agt gta aat tac atg      96
Glu Arg Val Thr Leu Thr Cys Arg Ala Ser Ser Ser Val Asn Tyr Met
            20                  25                  30

cac tgg tac cag cag aag cca gga tcc tcc ccc aaa gcc tgg att tat      144
His Trp Tyr Gln Gln Lys Pro Gly Ser Ser Pro Lys Ala Trp Ile Tyr
        35                  40                  45

gcc aca tcc aac ctg gct tct gga gtc cct gat cgc ttc agt gcc agt      192
Ala Thr Ser Asn Leu Ala Ser Gly Val Pro Asp Arg Phe Ser Ala Ser
    50                  55                  60

ggg tct ggg acc tct tac tct ctc aca atc agc aga gtg gag gct gaa      240
Gly Ser Gly Thr Ser Tyr Ser Leu Thr Ile Ser Arg Val Glu Ala Glu
65                  70                  75                  80

gat gct gcc act tat tac tgt cag cag tgg aga act aac cca cct acg      288
Asp Ala Ala Thr Tyr Tyr Cys Gln Gln Trp Arg Thr Asn Pro Pro Thr
                85                  90                  95

ttc ggt gct ggg acc aag ctg gag ctg aaa cgg                          321
Phe Gly Ala Gly Thr Lys Leu Glu Leu Lys Arg
            100                 105


<210>  5
<211>  107
<212>  PRT
<213>  Mus musculus

<400>  5

Gln Val Val Leu Ser Gln Ser Pro Ala Ile Leu Ser Ala Ser Pro Gly
1               5                   10                  15


Glu Arg Val Thr Leu Thr Cys Arg Ala Ser Ser Ser Val Asn Tyr Met
            20                  25                  30


His Trp Tyr Gln Gln Lys Pro Gly Ser Ser Pro Lys Ala Trp Ile Tyr
        35                  40                  45


Ala Thr Ser Asn Leu Ala Ser Gly Val Pro Asp Arg Phe Ser Ala Ser
    50                  55                  60


Gly Ser Gly Thr Ser Tyr Ser Leu Thr Ile Ser Arg Val Glu Ala Glu
65                  70                  75                  80


Asp Ala Ala Thr Tyr Tyr Cys Gln Gln Trp Arg Thr Asn Pro Pro Thr
                85                  90                  95


Phe Gly Ala Gly Thr Lys Leu Glu Leu Lys Arg
            100                 105

<210>  6
<211>  10
<212>  PRT
<213>  Artificial

<220>
<223>  complementarity determining region (CDR)


<220>
<221>  MISC_FEATURE
<222>  (1)..(10)
<223>  Denomination of CDR protein sequences in Kabat Nomenclature of
       NI-103 CDRH1

<400>  6

Gly Tyr Thr Phe Thr Glu Tyr Thr Met His
1               5                   10


<210>  7
<211>  17
<212>  PRT
<213>  Artificial

<220>
<223>  complementarity determining region (CDR)


<220>
<221>  MISC_FEATURE
<222>  (1)..(17)
<223>  Denomination of CDR protein sequences in Kabat Nomenclature of
       NI-103 CDRH2

<400>  7

Gly Val Asn Pro Tyr Asn Asp Asn Thr Ser Tyr Ile Arg Lys Leu Gln
1               5                   10                  15


Gly



<210>  8
<211>  13
<212>  PRT
<213>  Artificial

<220>
<223>  complementarity determining region (CDR)


<220>
<221>  MISC_FEATURE
<222>  (1)..(13)
<223>  Denomination of CDR protein sequences in Kabat Nomenclature of
       NI-103 CDRH3

<400>  8

Tyr Gly Gly Leu Arg Pro Tyr Tyr Phe Pro Met Asp Phe
1               5                   10

<210>  9
<211>  10
<212>  PRT
<213>  Artificial

<220>
<223>  complementarity determining region (CDR)

<220>
<221>  MISC_FEATURE
<222>  (1)..(10)
<223>  Denomination of CDR protein sequences in Kabat Nomenclature of
       NI-103 CDRL1

<400>  9

Arg Ala Ser Ser Ser Val Asn Tyr Met His
1               5                   10

<210>  10
<211>  7
<212>  PRT
<213>  Artificial

<220>
<223>  complementarity determining region (CDR)

<220>
<221>  MISC_FEATURE
<222>  (1)..(7)
<223>  Denomination of CDR protein sequences in Kabat Nomenclature of
       NI-103 CDRL2

<400>  10

Ala Thr Ser Asn Leu Ala Ser
1               5

<210>  11
<211>  9
<212>  PRT
<213>  Artificial

<220>
<223>  complementarity determining region (CDR)

<220>
<221>  MISC_FEATURE
<222>  (1)..(9)
<223>  Denomination of CDR protein sequences in Kabat Nomenclature of
       NI-103 CDRL3

<400>  11

Gln Gln Trp Arg Thr Asn Pro Pro Thr
1               5